Europäisches Patentamt

European Patent Office

Office européen des brevets

(11) Publication number: **0 086 362**
**B1**

(12) # EUROPEAN PATENT SPECIFICATION

(45) Date of publication of patent specification: **18.03.87**

(51) Int. Cl.⁴: **C 07 C 51/41**

(21) Application number: **83100651.5**

(22) Date of filing: **25.01.83**

(54) Granular metal soap product and process for producing metal soap.

(30) Priority: **26.01.82 US 342906**

(43) Date of publication of application:
**24.08.83 Bulletin 83/34**

(45) Publication of the grant of the patent:
**18.03.87 Bulletin 87/12**

(84) Designated Contracting States:
**AT BE CH DE FR GB IT LI NL SE**

(56) References cited:
**EP-A-0 017 827**
**DE-A-2 823 002**
**GB-A-1 406 526**
**US-A-3 519 571**
**US-A-3 803 188**
**US-A-4 060 535**

(73) Proprietor: **Mallinckrodt, Inc. (a Delaware corporation)**
**675 McDonnell Boulevard P.O. Box 5840**
**St. Louis Missouri 63134 (US)**

(72) Inventor: **Scully, Daniel F.**
**14646 Britannia Drive**
**Chesterfield Missouri (US)**
Inventor: **Fischer, Julius F.**
**721 N. Elizabeth**
**Ferguson Missouri (US)**
Inventor: **Nelson, Edgar N.**
**726 Decker Lane**
**Creve Coeur Missouri (US)**
Inventor: **Hudson, Richard**
**1602 Blueberry**
**Imperial Missouri (US)**
Inventor: **Roberts, Gregory A.**
**5921 Sir Edward Court**
**Florissant Missouri (US)**

(74) Representative: **Kraus, Walter, Dr. et al**
**Patentanwälte Kraus, Weisert & Partner**
**Thomas-Wimmer-Ring 15**
**D-8000 München 22 (DE)**

Courier Press, Leamington Spa, England.

## Description

The present invention relates to a process for producing metal soaps of a carboxylic acid containing between 8 and 22 carbon atoms by reaction of metal oxides, hydroxides and carbonates with carboxylic acids in the presence of water.

Metal soaps are used extensively in the manufacture of products constituted of plastics such as polyolefins, polyvinylchloride, acrylonitrile/-butadiene/styrene copolymers, reinforced polyester, polystyrene and impact resistant polystyrene. The soaps may serve as stabilizers and lubricants in plastic molding compounds and are also useful as mold release agents and rheological flow modifiers in the processing of plastics. In most plastics operations, though not all, granulated forms of metallic soaps function completely satisfactorily because the ultimate performance of the soap is independent of its particle size. As a consequence, the use of a granular product provides a significant advantage to the plastic processor or fabricator. Use of a granular soap eliminates air-born dust and thus greatly minimizes the chance of dust explosions and the nuisance of inhalation of the dust. Other benefits arise from reduced storage space requirements for and easier handling of granular metallic soaps in plant operations such as screw conveying, and from the better flow properties exhibited by the granular product when dispensed from hoppers or tote bins.

Metal soaps of fatty aids are long known to the art and several basic methods are available for their preparation. Alkali metal soaps are made by direct saponification of the acid or a glyceride thereof while the oldest known method for producing soaps of Group II metals is a double decomposition reaction in which a water-soluble (typically alkali metal) soap is initially formed, followed by addition of a Group II metal cation or compound which precipitates an insoluble soap of the Group II metal. The precipitate is recovered by filtration and the water evaporated to yield the final product.

Another known method for preparation of metal soaps is direct fusion in which melted fatty acids and oxides or hydroxides of metals are reacted at high temperature, followed by cooling and milling. In aqueous fusion, the metal oxide, hydroxide or carbonate is reacted with a fatty acid in an aqueous medium followed by filtration, drying, and milling.

US—A—2,899,232 describes a modified fusion technique in which a molten fatty acid is intimately mixed with a metal oxide, hydroxide or carbonate; and 0.5—3.0moles of water per mole of oxide or hydroxide is thereafter added to the mixture of fatty acid and metal compound. However, because the reaction of metal compound is highly exothermic, it proceeds almost instantaneously with a substantial increase in temperature, resulting in a hard fused product that is difficult to mill.

US—A—3,803,188 discloses a modification of the fusion process taught by the basic patent (US—A—2,899,232). According to US—A—2,899,232 a molten fatty acid is intimately mixed with a metal oxide, hydroxide or carbonate and 0.5—3.0 moles of water per mole of oxide or hydroxide is thereafter added to the mixture of fatty acid and metal compound. In US—A—3,803,188 the metal oxide or hydroxide is mixed with a molten fatty acid followed by the addition of relatively large proportions of water (3.5—40 moles/mole of oxide or hydroxide) which produces a fluffy and readily milled metal soap.

US—A—3,476,786 describes an anhydrous process in which flaked tallow acid is ground with a metal oxide, hydroxide or carbonate using ammonium carbonate as a catalyst. Although the reaction is slow, a fluffy powder eventually results.

US—A—4,060,535 discloses the use of various high shear mixers in grinding metal oxides, hydroxides, or carbonates together with powdered or flaked fatty acid in the presence of small quantities of water at a temperature below the melting point of the carboxylic acid component. White free-flowing powders are said to be obtained.

US—A—4,235,794 describes a process in which a metal soap granulate is formed by reacting an oxide, hydroxide, or carbonate of a metal compound with a fatty acid in an aqueous system at a temperature below the melting point of the fatty acid. The granulates thus formed must then be tray dried to remove water. Although the reference describes the addition of metal compound to the water/acid mixture, the metal compound is all added before the reaction is initiated, and the reaction is carried out in water solution. Moreover, this reference recommends that, if a relatively high acid to water ratio is used, "intensive or vigorous agitation and mixing is required to prevent caking of the granulate".

JA—A—55—136244 discloses a process for producing a granular metallic soap by mixing an aliphatic carboxylic acid with a metal oxide, hydroxide or carbonate and water, the water amounting to 15% of the total amount of the acid. The mixture is stirred in a pressure vessel and reacted under 1.1—1.3 atmospheres pressure at a temperature of 90—110°C. Vigorous agitation is employed and a very large particle size in the range of 0.2—2.0 millimeters is obtained.

Although a variety of methods are thus known to the art for the preparation of metallic soaps, essentially all involve premixing of the fatty acid and metal compound with subsequent reaction either by addition of water or under anhydrous conditions in the presence of a catalyst. For the most part, the reaction products are either very fine bulky powders or very coarse granules, such as those obtained in the Japanese published application process. Both the very fine and very coarse products are subject to dusting. Moreover, in many of these processes thermal degradation and darkening of the product due to high localized temperatures may result. Accordingly, a need has remained in the art for an improved process for producing granular substantially dust-free metal soaps.

The object of the present invention is the provision of an improved process of the preparation of metal soaps which produces a granular free-flowing material and substantially avoids thermal degradation of the soap. This process should be amenable to adjustment for errors in stoichiometry to provide positive control of the free acid content of the product and should produce a granular product of exceptionally high density. The reaction product should be dried with minimal energy input. The process should provide for the controlled production of different particle size grades of metal soap products for use in different applications. Finally a novel granular high density substantially dust free metal soap product should be provided according to the present invention.

According to the invention, the above-mentioned object is achieved by a process as described above which is characterized by the steps of preparing a mixture comprising said carboxylic acid and between 0.5 and 20 moles of water per mole of acid; slowly adding to said mixture a solid state metal compound comprising an oxide, hydroxide or carbonate of a metal selected from the group consisting of calcium, zinc, strontium, magnesium, lithium, sodium, potassium, barium and lead, whereby reaction between said acid and said metal compound is initiated and progresses as addition of said compound is carried out; and gently agitating the reacting mixture to produce a granular soap of said carboxylic acid and said metal.

The invention is further directed to a novel granular free flowing soap of calcium, zinc, strontium, magnesium, lithium, sodium, potassium, barium or lead and a carboxylic acid having 8—22 carbon atoms. The product is substantially dust-free and has a particle size distribution such that no more than about 20% by weight is retained on a 0.42 mm screen (40 mesh screen; U.S. sieve size) while no more than about 25% by weight passes through a 0.074 mm screen (200 mesh screen; U.S. sieve size). The particles are substantially spherical and the product has a bulk density of between 513 and 673 g/l (between 32 and 42 pounds/cubic foot) and a free carboxylic acid content of between 0.1 and 5% by weight.

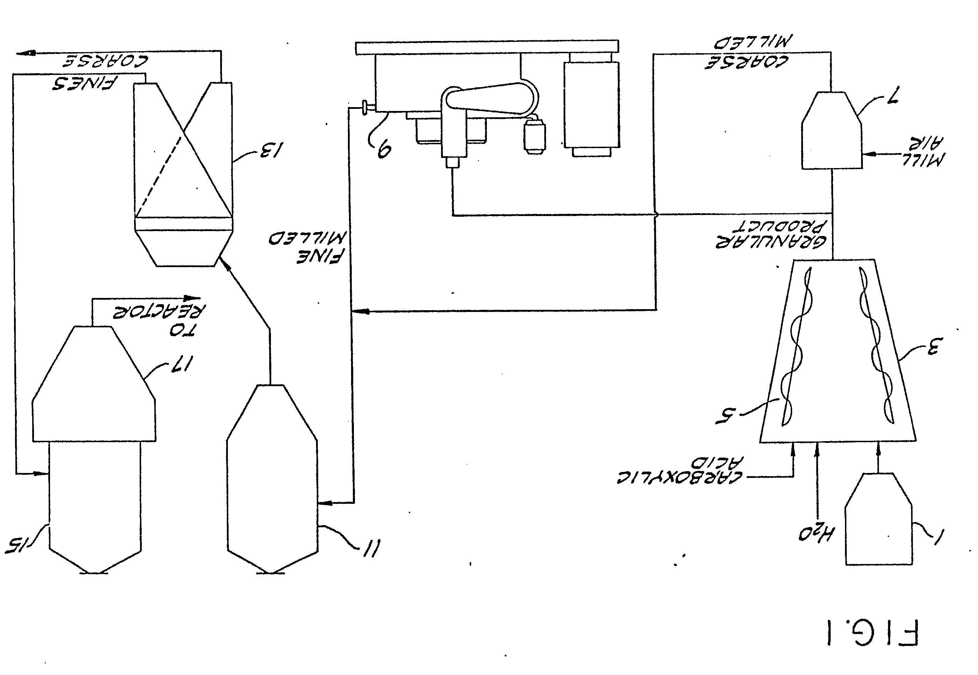

The single figure of the drawing is a simplified schematic flow diagram of the process of the invention.

In accordance with the present invention, it has been discovered that several major advantages can be achieved in the manufacture of metal soaps by altering the conventional procedure for the reaction step so that the metal oxide (or other metal compound) is added to a pre-mixture containing water and a fatty acid. Because only relatively small proportions of unreacted metal compound are present in the reaction mixture at any given time, positive control of the reaction rate is provided, thus avoiding the thermal degradation arising from rapid exothermic heating to high temperatures which has typically characterized prior art processes that have used water in the reaction system. Moreover, it has been found that the product of the reaction is a free-flowing granular product of exceptionally high bulk density. Its particle size is such that it may be readily coarse milled to provide an optimized particle size fraction that offers unique advantages in various applications, most particularly in powder metallurgy and plastic processing.

Illustrated in the drawing is a schematic flow sheet for carrying out the process of the invention. Shown at 3 is a conical reactor of the Nauta type which contains stirrers 5 which are driven in planetary fashion around the periphery of the reactor vessel. Other moderate shear mixers may be used such as, for example, Sigma mixers, Plow blenders and ribbon blenders. A charge hopper 1 contains a powdered metal compound which is charged slowly to reactor 3 after the mixture of carboxylic acid and water has been prepared therein. Product from the reactor may be milled either in a coarse mill 7 or a fine mill 9, and the milled product is collected in a milled product bag collector 11. From the collector 11 the milled product is delivered to an air classifier 13 which divides it into a coarse fraction constituting the product of the invention and a fine fraction which is collected in a recycled bag collector 15 and discharged into a recycle fine hopper 17 from which it may be recycled to the reactor.

Metal soaps of a wide variety of carboxylic acids containing 8—22 carbon atoms can be produced in accordance with the invention. Among the various acids which can be used are capric acid, lauric acid, myristic acid, palmitic acid, stearic acid, oleic, arachidic acid, behenic acid and abietic acid. The process of the invention is particularly advantageous for the manufacture of the metal soaps of commercially available hydrogenated tallow acid which comprises a mixture of palmitic and stearic acids. Other mixtures of acids containing 8—22 carbon atoms can also be used.

In accordance with the process of the invention, molten carboxylic acid and water are blended to provide a liquid feed mixture. Preferably the feed mixture contains between 0.5 and 20 moles water per mole of acid.

If necessary, it may also contain a surfactant for purposes of minimizing dusting during the reaction. A variety of surfactants may be used, for example the condensate of nonylphenol and ethylene oxide sold under the trade designation Igepal CO—630 by GAF Corporation. However, use of a surfactant is not normally necessary and, in fact, is preferably avoided since surfactants adversely affect the bulk density of the product and also retard drying.

Preparatory to the addition of metal compound, the mixture is preferably heated to a temperature in excess of the melting point of the carboxylic acid. Optionally, water and acid are heated prior to introduction into the reactor so that the mixture as initially prepared has a temperature in the range of the melting point of the acid to 98°C. By introduction of steam into a jacket or coils associated with the reactor, the initial temperature of the mixture can be maintained or increased. When the addition of metal compound is commenced, the liquid mixture in the reactor optionally can be agitated vigorously so as to achieve uniform initial dispersion of the metal compound. However, this is not essential, and entirely satisfactory results can be achieved if only gentle agitation is maintained throughout the reaction cycle.

The metal compound charged to the reactor comprises an oxide, hydroxide, or carbonate of calcium, zinc, strontium, magnesium, lithium, sodium, potassium, barium, or lead. In the case of zinc, zinc oxide is most conveniently used, while in the case of calcium, calcium hydroxide is preferred. Mixtures of two or more metal compounds can also be used. By using metal compounds of two or more metals, mixed carboxylic acid soaps may be prepared.

In the case of zinc oxide, a catalyst for the reaction is preferably included in the liquid mixture to which the metal compound is added. Preferably the catalyst is a chelating acid such as citric acid, tartaric acid, or glycolic acid. Zinc salts of these acids or of other weak acids (for example zinc acetate) can also be used. It is believed that the catalyst acid reacts with the metal compound to produce an intermediate which thereafter reacts with the carboxylic acid to produce the soap and regenerate the catalyst.

The metal compound is added slowly to the liquid mixture at such rate as to control the temperature of the reacting mixture in the range of between the melting point of the carboxylic acid and about 98°C. Higher temperatures can be used by operating under pressure but this offers little or no advantage and is not preferred. Preferably the bulk of the reaction is carried out at temperatures in the range of 70°C. If necessary, cooling water may be used through a jacket or coil associated with the reactor to maintain the temperature within the desired range. However, because the rate of generation of exothermic heat may be controlled by the rate of addition of metal compound, and the heat gain from the reaction is increasingly offset by steam generation as the temperature rises, the use of cooling water is not normally required. Thus, where the liquid mixture has been preheated to a temperature in a lower portion of the range, the temperature may typically rise during the course of a metal compound addition to a temperature near the upper end of the range, where it tends to stabilize. Thus, for example, if the liquid mixture is preheated to a temperature in the range of 60—70°C, the temperature of the reacting mixture can be permitted to rise during the reaction to the range of 90—98°C, where it levels off.

Although the metal compound is preferably added continuously, it may alternatively be added in a series of small increments. In either case, addition is typically carried out over a period of 10 minutes to 2 hours, normally 0.5—2 hours.

Whether the metal compound is added continuously or incrementally, the proportion of unreacted metal compound in the mixture at any given time is relatively small and the rate of reaction and generation of exothermic heat are thereby controlled. Thus, not only is the bulk of the reaction mixture maintained within the desired temperature range, but localized hot spots and the thermal degradation associated with them are effectively avoided. In the case of those soaps containing a colorless cation, undesirable darkening of the product is thereby avoided.

Addition of metal compound to the water/carboxylic acid liquid mixture also permits precise control of the stoichiometry of the reaction. More particularly, the addition of metal compound can be terminated when analysis shows that the desired final free acid content has been or will be reached. Total reaction time may typically be 3 to 5 hours. The end point may be readily determined by extracting free fatty acid from the reaction mixture with acetone, and titrating with NaOH. It has been found that final free acid content is an important parameter since the particle size and performance properties of the granular product are optimized when the final free acid content is in the range of 0.1 to 5%, preferably 0.5 to 2%, by weight.

Although the bulk of the water is mixed with the carboxylic acid prior to metal compound addition, it is sometimes advantageous to add some water during the course of the reaction. In any event, it is important that the concentration of water in the reaction mixture not be allowed to fall below 1—4% by weight or the reaction will cease. As noted above, steam evolves from the reacting mixture as its temperature rises due to exothermic heat. The mass transfer driving force for water evolution is further increased where the head space in the reactor is air swept to remove dust that might otherwise present an explosion hazard. Thus, even though water is a product of the reaction, it is possible to drive enough water out of the reacting mixture so that the total water content falls below the desired range. If a sample taken at the end of the normal reaction period shows high fatty acid content, it indicates that the batch has run dry and water is added to cause the reaction to be completed. Alternatively, the reacting mixture may be monitored and incremental additions of water made if the water content falls below the 1—4% range.

By slow and gentle agitation of the reacting mixture, a coarse granular product is obtained whose particle size is typically of such a distribution that approximately 40% is retained on a 2.00 mm screen, 50% passes through a 0.42 mm screen, and only 10—15% passes through a 0.074 mm screen. As noted, vigorous agitation may be used initially to obtain uniform initial dispersion of the metal compound but once the reaction has proceeded to the point that a significant increase in viscosity may be observed, only slow agitation should be used. Usually the reaction mass proceeds through several stages. Initially it has the appearance of pigment dispersed in a fatty acid, but soon becomes creamy and increases in viscosity until it has the consistency of a mortar. When the viscosity has increased to such mortar-like consistency, any high speed agitation should be stopped and the remainder of the metal compound added under gentle but thorough agitation conditions.

As a result of the controlled addition of metal compound to the water/carboxylic acid mixture, the overall amounts of water used in the reaction are modest and the proportion of water in the reaction mixture at the conclusion of the reaction is minimized. Thus, the product of the reaction can be dried simply by subjecting it to a vacuum, preferably a relatively high vacuum of at least 0.68 bar (20 inches of mercury). Under such vacuum conditions, and with the reaction product at a temperature in excess of approximately 90°C as a result of autogenous reaction heat, the residual moisture is driven off without any significant input of external energy. In the drying step, the temperature of the soap drops to about 65°C. The process of the invention thus provides major advantages from the standpoint of energy consumption. Because of the control afforded by the technique of adding the metal compound to the water/carboxylic acid mixture, maximum advantage is taken of the exothermic reaction heat and overall consumption of fuel energy is very low as compared to most prior art processes.

The granulated product of the reaction step is substantially non-dusting and highly suited for use in the various known applications for metal soaps. The product is constituted of substantially spherical particles or aggregates, possesses rather excellent structural integrity, and exhibits a surprisingly high bulk density of 320—721 g/l (20—45 pounds/cubic foot). As noted, the particle size is typically 85—90% plus 0.074 mm and any minus 0.074 mm fines can be sieved free of the granular grade product and recycled through the reaction step.

Although the product of the reaction step has advantageous properties, it has been found that a product having even more favorable properties can be produced by coarse milling of the granular reaction product and air-classifying the coarse milled material. In the flow sheet as illustrated in the drawing, the granular reaction product may also be delivered to a fine mill 9 where it is-reduced to a fine powder that may be desired in certain applications.

In the coarse milling step, the granular product of the reaction is reduced so that it is substantially all minus 2.00 mm and 90—99% minus 0.42 mm sieve size, but the proportion of plus 0.074 mm material (85—95%) is not significantly decreased. For this purpose, a variety of conventional coarse milling equipment can be used, with a hammer mill being preferred. The coarse milled product is air conveyed from the coarse mill to a bag collector from whence it is sent to an air classifier for separation of the fines that may be contained in the granular reaction product or produced in the coarse milling step. For the air classification, an Alpine spiral air classifier is preferably used, but a variety of other air classification equipment capable of making the desired cut can also be utilized. As shown in the drawing, fines from the air classifier are collected in a fines bag collector and dumped in from the collector to a recycle fines hopper, and may thereafter be recycled to the reactor.

The coarse fraction from the air classifier is a unique product that has been found to be highly advantageous in plastics and other applications for metal soaps. Its particle size distribution is such that no more than about 20%, typically 1—20%, is retained on a 0.42 mm screen while no more than about 25% by weight, preferably 5 to 15%, passes through a 0.074 mm screen. This product is constituted of substantially spherical particles and exhibits an unusually high bulk density in the range of 513—673 g/l. It has a free carboxylic acid content in the range of 0.1 to 5%, preferably 0.5—2%, by weight.

In the fine milling step, the product produced is typically all minus 0.044 mm, but a minus 0.074 mm, plus 0.037 mm product may alternatively be produced. When used in powder metallurgy it has been found to afford major improvement in flow characteristics of the powdered metal as compared to previously available metal soaps. As the fine mill, a Mikropul ACM 60 impact mill with internal air classification is preferably used, but other conventional fine milling equipment may also be suitable.

The following examples illustrate the invention:

Example 1

Granulated calcium stearate was produced in a conical shaped Nauta mixer reactor (JH Day Company) Model MBX 105 having a rotating screw (45—90 rpm) attached to an orbit arm (1—3 rpm) and provided with a delumper. Hydrogenated tallow fatty acid containing approximately 65% by weight stearic acid and 35% by weight palmitic acid (113 kg; 0.905 mole) was charged to the Nauta mixer reactor. To this was added water (11.3 kg; 1.38 moles) and the liquid mixture was heated to a temperature of 60°C, after which calcium hydroxide (15.9 kg; 0.472 moles) was added steadily to the acid/water mixture over a period of thirty minutes. The reacting mixture was initially stirred vigorously to disperse the calcium hydroxide but, as the mixture approached mortar-like consistency, the intensity of agitation was reduced to provide gentle but thorough agitation. As reaction progressed, the temperature was allowed to rise to the range of 90—95°C. Vacuum was applied to remove residual water and cool the product to 65°C. The final product had a bulk density of 753 g/l, a free fatty acid content of 0.78% and a moisture content of 3.0%. Sifting to remove −0.074 mm material produced an 82% yield of dust-free +0.074 mm granular material.

### Example 2

To a reactor of the type described in Example 1 were added hydrogenated tallow fatty acid containing approximately 65% by weight stearic acid and 35% by weight palmitic acid (113 kg; 0.904 mole), water (11.3 kg; 1.38 moles) and fine (−0.074 mm) calcium stearate recovered from the sifting step of Example 1. This mixture was heated to a temperature of 65°C after which powdered calcium hydroxide (15.9 kg; 0.472 moles) was added steadily to the mixture over a period of 30 minutes. Gentle agitation was maintained from and after the point at which the reaction mixture attained a mortar-like consistency. Reaction was complete in one hour after addition of the calcium hydroxide, producing an 86% yield of granular product after removal of fines through a 0.074 mm screen.

### Example 3

To a reactor of the type described in Example 1 were introduced hydrogenated tallow fatty acid (113 kg; 0.905 moles) and water (11.3 kg; 1.38 moles). The liquid mixture was heated to 70°C, after which powdered zinc oxide (0.49 moles) was added slowly over a period of one hour. During zinc oxide addition the temperature of the reaction mass rose to 95—98°C. Gentle agitation was maintained from and after the point that the reacting mixture achieved a mortar-like consistency. After one half to one hour of gentle agitation, a product containing less than 1% by weight free fatty acid was obtained. After sifting for removal of fines, a 50% yield of granular (+0.074 mm) zinc stearate was obtained.

### Example 4

To a reactor of the type described in Example 1 were added hydrogenated tallow fatty acid (0.905 moles), water (18.1 kg; 2.2 moles) and zinc stearate fines (56.7 kg) recycled from the preparation of Example 3. Powdered zinc oxide (18.4 kg; 0.49 moles) was added slowly to this mixture over a period of one hour with gentle agitation. After a period of 30 to 60 minutes at 95°C, reaction was complete, producing a granular fraction of 68% plus 0.074 mm material having a bulk density of 609 g/l.

Granular soaps of the type prepared in the above examples are coarse milled in a hammermill of the type equipped with a rotor containing stirrup hammers, a smooth mill chamber liner and a screen. Grinding or milling is accomplished by the impingement of particles against the hammers and the tip speed of the hammers can be varied to control the amount of grinding. Ground material is thrown out of the mill chamber through the screen by the centrifugal force created by the grinding rotor. The screen preferably used is a herring-bone type with 0.32 cm slots. However, other types of screens (round hole or straight slot) can also be used to control the maximum size of particles leaving the mill chamber.

The milled material is air conveyed to a classifier in which a recirculating air stream encounters an opposing centrifugal force created by a fan, thereby producing a separation of large and small particles. Separation is thus enhanced by multi-stage fractionation. The cut point in this equipment is adjusted to provide, in the coarse fraction emanating therefrom, the particle size noted above for the product of the invention.

If desired, granular product of the invention may also be fine milled using a hammer mill of conventional construction.

**Claims**

1. A process for producing metal soaps of a carboxylic acid containing between 8 and 22 carbon atoms by reaction of metal oxides, hydroxides and carbonates with carboxylic acids in the presence of water, characterized by the steps of:

preparing a mixture comprising said carboxylic acid and between 0.5 and 20 moles of water per mole of acid;

slowly adding to said mixture a solid state metal compound comprising an oxide, hydroxide or carbonate of a metal selected from the group consisting of calcium, zinc, strontium, magnesium, lithium, sodium, potassium, barium and lead, whereby reaction between said acid and said metal compound is initiated and progresses as addition of said compound is carried out; and

gently agitating the reacting mixture to produce a granular soap of said carboxylic acid and said metal.

2. A process as set forth in claim 1 wherein the rate of addition of said metal compound is controlled so as to control the temperature of the reacting mixture at between the melting point of said acid and about 98°C, thereby minimizing thermal degradation and darkening of the product.

3. A process as set forth in claim 1 wherein the water content of the reacting mixture is maintained at between 1 and 4% by weight.

4. A process as set forth in claim 3 wherein minor amounts of water are added to the reacting mixture to maintain said water content.

5. A process as set forth in claim 1 wherein said mixture further comprises a catalyst comprising an acid which reacts with said metal compound to produce an intermediate, said intermediate thereafter reacting with said carboxylic acid to produce said soap and regenerate said catalyst.

6. A process as set forth in claim 5 wherein said catalyst comprises a chelating acid.

7. A process as set forth in claim 6 wherein said chelating acid is selected from the group consisting of citric acid, tartaric acid, and glycolic acid.

8. A process as set forth in claim 1 wherein said mixture further comprises a nonionic surfactant.

9. A process as set forth in claim 1 wherein said agitation is terminated when the free-carboxylic acid content of the reacting mixture is between 0.1 and 5% by weight.

10. A process as set forth in claim 1 wherein upon completion of the reaction, the reaction product is subjected to a vacuum for removal of the free water content.

11. A process as set forth in claim 10 wherein the reaction product is subjected to a vacuum of at least 0.68 bar.

12. A process as set forth in claim 10 wherein the granular product is coarse milled to reduce the particle size of the granular product so that it is substantially 100% by weight minus 2.00 mm (10 mesh), 1—10% by weight plus 0.42 mm (40 mesh) and 85—95% by weight plus 0.074 mm (200 mesh, U.S. sieve size).

13. A process as set forth in claim 12 wherein the coarse milled product is air classified to produce a coarse fraction whose particle size is such that no more than about 20% by weight is plus 0.42 mm (40 mesh) and at least 85% by weight is plus 0.074 mm (200 mesh, U.S. sieve size).

14. A process as set forth in claim 1 wherein a mixture of metal soaps is produced by adding to said mixture of carboxylic acid and water a plurality of said metal compounds, said plurality including compounds of two or more of said metals.

15. A process as set forth in claim 1 wherein said metal compound comprises zinc oxide.

16. A process as set forth in claim 1 wherein said metal compound comprises calcium hydroxide.

17. A process as set forth in claim 1 wherein said metal compound comprises magnesium oxide.

18. A process as set forth in claim 1 wherein said metal compound comprises sodium hydroxide.

19. A process as set forth in claim 1 wherein said metal compound comprises potassium hydroxide.

20. A process as set forth in claim 1 wherein said metal compound comprises a mixture of calcium hydroxide and zinc oxide.

21. A granular free flowing soap of a carboxylic acid having between 8 and 22 carbon atoms and a metal selected from the group consisting of calcium zinc, strontium, magnesium, lithium, sodium, potassium, barium and lead and mixtures thereof, the particles of said soap being substantially spherical and having a particle size distribution such that no more than about 20% by weight thereof is retained on a 0.42 mm screen (40 mesh screen; U.S. sieve size) while no more than about 25% by weight passes through a 0.074 mm screen (200 mesh screen; U.S. sieve size), said granular soap having a bulk density of between 513 and 673 g/l.

**Revendications**

1. Procédé de production de savons métalliques d'un acide carboxylique contenant entre 8 et 22 atomes de carbone par réaction d'oxydes, d'hydroxydes et de carbonates métalliques avec des acides carboxyliques en présence d'eau, caractérisé par les étapes de:

préparation d'un mélange comprenant cet acide carboxylique et entre 0,5 et 20 moles d'eau par mole d'acide;

lente addition à ce mélange d'un composé métallique à l'état solide comprenant un oxyde, un hydroxyde ou uncarbonate d'un métal choisi dans le groupe consistant en calcium, zinc, strontium, magnésium, lithium, sodium, potassium, baryum et plomb, si bien que la réaction entre l'acide et le composé métallique est initiée et se déroule au fur et à mesure de l'addition du composé; et

agitation douce du mélange réactionnel pour produire un savon granulé de l'acide carboxylique et du métal.

2. Procédé selon la revendication 1 dans lequel le taux d'addition du composé métallique est contrôlé de façon à maintenir la température du mélange réactionnel entre le point d'ébullition de l'acide et environ 98°C, ce qui minimise ainsi la dégradation thermique et le noircissement du produit.

3. Procédé selon la revendication 1, dans lequel la teneur en eau du mélange réactionnel est maintenue entre 1 et 4% en poids.

4. Procédé selon la revendication 3, dans lequel des quantités mineures d'eau sont ajoutées au mélange réactionnel pour maintenir cette teneur en eau.

5. Procédé selon la revendication 1, dans lequel le mélange comprend de plus, un catalyseur comprenant un acide qui réagit avec ce composé métallique pour produire un intermédiaire, cet intermédiaire réagissant ensuite avec l'acide carboxylique pour produire le savon et régénérer le catalyseur.

6. Procédé selon la revendication 5, dans lequel le catalyseur comprend un acide chélatant.

7. Procédé selon la revendication 6, dans lequel l'acide chélatant est choisi dans le groupe consistant en l'acide citrique, l'acide tartrique et l'acide glycolique.

8. Procédé selon la revendication 1, dans lequel le mélange comprend de plus un tensioactif non ionique.

9. Procédé selon la revendication 1, dans lequel l'agitation est arrêtée lorsque la teneur en acide carboxylique libre du mélange réactionnel est entre 0,1 et 5% en poids.

10. Procédé selon la revendication 1, dans lequel le produit de réaction est soumis à un vide pour éliminer la teneur en eau libre, lorsque la réaction est achevée.

11. Procédé selon la revendication 10, dans lequel le produit de réaction est soumis à un vide d'au moins 0,68 bar.

12. Procédé selon la revendication 10, dans lequel le produit granulé est broyé grossièrement pour réduire la dimension des particules du produit granulé de telle sorte qu'il y ait pratiquement 100% en poids de moins de 2,00 mm (10 mesh), 1 à 10% en poids de plus de 0,42 mm (40 mesh) et 85 à 95% en poids de plus de 0,074 mm (200 mesh, dimension des tamis US).

13. Procédé selon la revendication 12, dans lequel le produit broyé grossièrement est criblé à l'air pour produire une fraction grossière dont la dimension particulaire est telle qu'il n'y ait pas plus d'environ 20% en poids de plus de 0,42 mm (40 mesh) et au moins 85% en poids de plus de 0,074 mm (200 mesh, dimension des tamis US).

14. Procédé selon la revendication 1, dans lequel on produit un mélange de savons métalliques en ajoutant au mélange d'acide carboxylique et d'eau, un certain nombre de ces composés métalliques, ce certain nombre comprenant des composés de deux ou plusieurs de ces métaux.

15. Procédé selon la revendication 1, dans lequel le composé métallique comprend l'oxyde de zinc.

16. Procédé selon la revendication 1, dans lequel le composé métallique comprend l'hydroxyde de calcium.

17. Procédé selon la revendication 1, dans lequel le composé métallique comprend l'oxyde de magnésium.

18. Procédé selon la revendication 1, dans lequel le composé métallique comprend l'hydroxyde de sodium.

19. Procéde selon la revendication 1, dans lequel le composé métallique comprend l'hydroxyde de potassium.

20. Procédé selon la revendication 1, dans lequel le composé métallique comprend un mélange d'hydroxyde de calcium et d'oxyde de zinc.

21. Savon granulé s'écoulant librement d'un acide carboxylique ayant entre 8 et 22 atomes de carbone et d'un métal choisi dans le groupe consistant en calcium, zinc, strontium, magnésium, lithium, sodium, potassium, baryum et plomb et leurs mélanges, les particules de ce savon étant pratiquement sphériques et ayant une distribution de dimension particulaire telle qu'il n'y ait pas plus de 20% en poids de celles-ci qui sont retenues sur un tamis de 0,42 mm (tamis de 40 mesh, dimension des tamis US), tandis qu'il n'y a pas plus de 25% en poids de celles-ci qui traversent un tamis de 0,074 mm (tamis de 200 mesh, dimension des tamis US), ce savon granulé ayant une densité en vrac entre 513 et 673 g/l.

**Patentansprüche**

1. Verfahren zur Herstellung von Metallseifen einer Carbonsäure mit zwischen 8 und 22 Kohlenstoffatomen durch Umsetzung von Metalloxiden, Hydroxiden und Carbonaten mit Carbonsäuren in Gegenwart von Wasser, gekennzeichnet durch die folgenden Stufen:

Herstellung eines Gemisches, das die genannte Carbonsäure und zwischen 0,5 und 20 Mol Wasser pro Mol Säure enthält;

langsame Zugabe einer festen Metallverbindung, umfassend ein Oxid, Hydroxid oder Carbonat eines Metalls, ausgewählt aus der Gruppe, bestehend aus Calcium, Zink, Strontium, Magnesium, Lithium, Natrium, Kalium, Barium und Blei, zu dem genannten Gemisch, wodurch die Reaktion zwischen der genannten Säure und der genannten Metallverbindung initiiert wird und fortschreitet, wie die Zugabe der genannten Verbindung durchgeführt wird, und

mäßiges Durchbewegen bzw. Rühren des Reaktionsgemisches, um eine kornförmige Seife aus der genannten Carbonsäure und dem genannten Metall herzustellen.

2. Verfahren nach Anspruch 1, dadurch gekennzeichnet, daß man die Geschwindigkeit der Zugabe der genannten Metallverbindung so kontrolliert, daß die Temperatur des reagierenden Gemisches auf zwischen den Schmelzpunkt der genannten Säure und etwa 98°C eingestellt wird, wodurch eine thermische Zersetzung und ein Dunkelwerden des Produkts minimalisiert werden.

3. Verfahren nach Anspruch 1, dadurch gekennzeichnet, daß man den Wassergehalt des reagierenden Gemisches auf zwischen 1 und 4 Gew.-% aufrechterhält.

4. Verfahren nach Anspruch 3, dadurch gekennzeichnet, daß man geringfügige Mengen von Wasser zu dem reagierenden Gemisch zusetzt, um den genannten Wassergehalt aufrechtzuerhalten.

5. Verfahren nach Anspruch 1, dadurch gekennzeichnet, daß das genannte Gemisch weiterhin einen Katalysator, umfassend eine Säure, enthält, der sich mit der genannten Metallverbindung unter Erzeugung eines Zwischenprodukts umsetzt, wobei das genannte Zwischenprodukt sich hiernach mit der genannten Carbonsäure unter Erzeugung der genannten Seife und Regenerierung des genannten Katalysators umsetzt.

6. Verfahren nach Anspruch 5, dadurch gekennzeichnet, daß der genannte Katalysator eine chelatisierende Säure umfaßt.

7. Verfahren nach Anspruch 6, dadurch gekennzeichnet, daß die genannte chelatisierende Säure aus der Gruppe, bestehend aus Zitronensäure, Weinsäure und Glykolsäure, ausgewält ist.

8. Verfahren nach Anspruch 1, dadurch gekennzeichnet, daß das genannte Gemisch weiterhin ein nichtionogenes oberflächenaktives Mittel enthält.

9. Verfahren nach Anspruch 1, dadurch gekennzeichnet, daß man das genannte Durchbewegen bzw. Rühren beendet, wenn der Gehalt an freier Carbonsäure des reagierenden Gemisches zwischen 0,1 und 5 Gew.-% liegt.

10. Verfahren nach Anspruch 1, dadurch gekennzeichnet, daß man nach Beendigung der Reaktion das Reaktionsprodukt hierauf einem Vakuum zur Entfernung des freien Wassergehalts aussetzt.

11. Verfahren nach Anspruch 10, dadurch gekennzeichnet, daß man das Raktionsprodukt einem Vakuum von mindestens 0,68 bar aussetzt.

12. Verfahren nach Anspruch 10, dadurch gekennzeichnet, daß man das kornförmige Produkt grob vermahlt, um die Teilchengröße des kornförmigen Produkts so zu verringern, daß es im wesentlichen aus 100 Gew.-% minus 2,00 mm (10 mesh), 1 bis 10 Gew.-% plus 0,42 mm (40 mesh) und 85 bis 95 Ges.-% plus 0,074 mm (200 mesh, US-Siebgröße) besteht.

13. Verfahren nach Anspruch 12, dadurch gekennzeichnet, daß man das grobgemahlene Produkt luftklassiert, um eine grobe Fraktion herzustellen, deren Teilchengröße so ist, daß nicht mehr als etwa 20 Gew.-% plus 0,42 mm (40 mesh) sind und daß mindestens 85 Gew.-% plus 0,074 mm (200 mm, US-Siebgröße) sind.

14. Verfahren nach Anspruch 1, dadurch gekennzeichnet, daß man ein Gemisch von Metallseifen in der Weise herstellt, daß man zu dem genannten Gemisch aus Carbonsäure und Wasser eine Vielzahl der genannten Metallverbindungen gibt, wobei die genannte Vielzahl Verbindungen von zwei oder mehreren der genannten Metalle einschließt.

15. Verfahren nach Anspruch 1, dadurch gekennzeichnet, daß die genannte Metallverbindung Zinkoxid umfaßt.

16. Verfahren nach Anspruch 1, dadurch gekennzeichnet, daß die genannte Metallverbindung Calciumhydroxid umfaßt.

17. Verfahren nach Anspruch 1, dadurch gekennzeichnet, daß die genannte Metallverbindung Magnesiumoxid umfaßt.

18. Verfahren nach Anspruch 1, dadurch gekennzeichnet, daß die genannte Metallverbindung Natriumhydroxid umfaßt.

19. Verfahren nach Anspruch 1, dadurch gekennzeichnet, daß die genannte Metallverbindung Kaliumhydroxid umfaßt.

20. Verfahren nach Anspruch 1, dadurch gekennzeichnet, daß die genannte Metallverbindung ein Gemisch aus Calciumhydroxid und Zinkoxid umfaßt.

21. Kornförmige freifließende Seife aus einer Carbonsäure mit zwischen 8 und 22 Kohlenstoffatomen und einem Metall, ausgewählt aus der Gruppe, bestehend aus Calcium, Zink, Strontium, Magnesium, Lithium, Natrium, Kalium, Barium und Blei und Gemischen davon, wobei die Teilchen der genannten Seife im wesentlichen kugelförmig sind und eine derartige Teilchengrößenverteilung haben, daß nicht mehr als etwa 20 Gew.-% davon auf einem 0,42-mm-Sieb (40-mesh-Sieb, US-Siebgröße) zurückbehalten werden, während nicht mehr als etwa 25 Ges.-% durch ein 0,074-mm-Sieb (200-mesh-Sieb, US-Siebgröße) hindurchgehen, wobei die genannte kornförmige Seife eine Schüttdichte von zwischen 513 und 673 g/l hat.

# FIG. I

0 086 362